# NEUE EUROPÄISCHE PATENTSCHRIFT

(11) **EP 1 222 910 B2**
(45) Veröffentlichungstag und Bekanntmachung des Hinweises auf die Entscheidung über den Einspruch: **02.07.2014**
(45) Hinweis auf die Patenterteilung: 19.05.2010
(21) Anmeldenummer: 02000118.6
(22) Anmeldetag: 03.01.2002
(51) Int. Cl.: A61K 6/083

(54) **Dentalmaterialien auf der Basis polyfunktioneller Amide**
Polyfunctional amide containing dental material
Matériau dentaire contenant des amides polyfonctionnels

(30) Priorität: 15.01.2001 DE 10101523
(43) Veröffentlichungstag der Anmeldung: 17.07.2002
(73) Patentinhaber: Ivoclar Vivadent AG, 9494 Schaan (LI)
(72) Erfinder: Moszner, Norbert, Prof. Dr., 9493 Mauren (LI); Zeuner, Frank, Dr., 9488 Schellenberg (LI); Rheinberger, Volker, Dr., 9590 Vaduz (LI); Angermann, Jörg, Dr., 7320 Sargans (CH); Völkel, Thomas, Dr., 88138 Sigmarszell (DE)
(74) Vertreter: UEXKÜLL & STOLBERG

(56) Entgegenhaltungen:
- WO-A-93/12759
- DE-A- 2 211 128
- DE-A- 19 648 282
- FERRUTI P ET AL: "RECENT RESULTS ON FUNCTIONAL POLYMERS AND MACROMONOMERS OF INTERESTAS BIOMATERIALS OR FOR BIOMATERIAL MODIFICATION" BIOMATERIALS, ELSEVIER SCIENCE PUBLISHERS BV., BARKING, GB, Bd. 15, Nr. 15, 1994, Seiten 1235-1241, XP001053477 ISSN: 0142-9612

## Beschreibung

Die vorliegende Erfindung betrifft Dentalmaterialien auf der Basis von polyfunktionellen, polymerisierbaren Amiden.

Monofunktionelle Amide der Acryl- bzw. Methacrylsäure finden in unterschiedlichen Bereichen der Zahnmedizin Anwendung. So offenbart die GB 1,039,750 Mischungen aus Methylmethacrylat und Acryl- oder Methacrylamid, wobei die Amide eine freie Hydroxyl-oder Ethergruppe enthalten. Als Vernetzer werden Verbindungen mit mindestens zwei olefinisch polymerisierbaren Doppelbindungen verwendet, wie z.B. Glycoldiacrylat, Dinvinylbenzol und Glycerin-tri(meth)acrylat. Die Mischungen sollen sich besonders zur Herstellung dentaler Prothesen eignen.

Aus der DE 23 16 603, der US 3,926,870 und der US 5,011,868 sind Gebißhaftmittel auf der Basis von Mischpolymerisaten von Acrylamiden bekannt.

Die US 3,660,343 offenbart dentale Adhäsive und Füllmaterialien die ein thermisch härtbares Epoxyharz und N-3-Oxokohlenwasserstoff substituiertes Acrylamid enthalten.

Aus der EP 0 394 792 A1 sind Formylpiperazingruppen enthaltende (Meth)acrylsäureester und (Meth)acrylsäureamide bekannt, die sich zur Herstellung von Adhäsiven und Füllmaterialien für den Dentalbereich eignen sollen.

Kitoh et al., J. Appl. Polym. Sci. 39 (1990) 103 und J. Appl. Polym. Sci. 51 (1994) 2021, haben den Einfluß von N-Substituenten auf das Haftungsvermögen von Methacrylamiden an Zahnschmelz und Dentalkeramiken untersucht.

Die WO 93/12759 offenbart wasserbasierte Amalgamadhäsive, die Wasser, säurereaktiven Füllstoff, wassermischbares saures Polymer, eine ethylenisch ungesättigte Komponente, wasserlösliches Reduktionsmittel, wasserlösliches Oxidationsmittel und gegebenenfalls Photoinitiator enthalten. Die ethylenisch ungesättige Komponente kann in Form von Monomeren wie Methylenbisacrylamid vorliegen.

Die DE 196 48 282 A1 offenbart säurestabile Borate, die sich als Initiatoren für die Photopolymerisation von säuregruppenhaltigen photopolymerisierbaren Zusammensetzungen eignen sollen.

Aus der DE 22 11 128 A1 sind Einbettmassen zur Herstellung von Zahnprothesen nach dem Kunststoffgießverfahren bekannt, die Acrylamid, N,N'-Methylenbisacrylamid, Initiator, Wasser und Dimethylaminopropionitril enthalten.

Ferner wurden monofunktionelle Acryl- bzw. Methacrylamide zur Synthese von polymeren Precursoren für Substrate mit Bleichwirkung eingesetzt (US 5,560,749).

Produkte, die durch vernetzende Copolymerisation von Acrylamiden mit Bis-[acryloylamino]-methan, zugänglich sind, können ein Vielfaches ihres Eigengewichts an Wasser aufnehmen und werden technisch als Superabsorber eingesetzt, beispielsweise zur Bodenverbesserung und -verfestigung, zur Herstellung von Inkontinenzartikeln oder als stationäre Phase in der Elektrophorese (W. M. Kulicke, Polymerisation von Acrylamiden und Methacrylamiden, In: Methoden der organischen Chemie (Houben-Weyl), Bd. E20/2, G. Thieme-Verlag, Stuttgart-New York 1987, 1176ff.).

Die WO 95/27008 offenbart Hilfsstoffe für die Papierherstellung die durch Reaktion eines Polyamins mit einem Vernetzer wie beispielsweise Bis(meth)acrylamid in einer wäßrigen Polyollösung hergestellt werden.

Die Verwendung polyfunktioneller (Meth)acrylamide für die Herstellung von Dentalmaterialien wurde bisher nicht beschrieben.

Der Erfindung liegt die Aufgabe zugrunde, Dentalmaterialien mit verbesserten Eigenschaften bereitzustellen, d.h. insbesondere von Dentalmaterialien mit hoher Hydrolysebeständigkeit

Diese Aufgabe wird durch Dentalmaterialien gelöst, die ein Amid der allgemeinen Formel BXₙ, in der
- B: für einen n-fach mit der Gruppe X substituierten Kohlenwasserstoffrest mit 2 bis 50 Kohlenstoffatomen steht, der eine oder mehrere der Gruppen O, S, NH, CO-NH, NH-CO, NH-CO-O, O-CO-NH und/oder NH-CO-NH enthalten kann, wobei
- B =: (1) eine gesättigte, lineare oder verzweigte aliphatische Gruppe mit 2 bis 15, insbesondere 2 bis 10 Kohlenstoffatomen, die eine oder zwei der Gruppen S, NH und insbesondere O, NH-CO-O oder O-CO-NH enthalten kann,
(2) eine cycloaliphatische Gruppe mit 6 oder 15 Kohlenstoffatomen,
(3) ein aromatischer oder nicht-aromatischer heterocyclischer Rest mit 3 bis 10 Kohlenstoffatomen und 1 bis 3 Heteroatomen,
(4) ein aromatischer Rest mit 6 bis 12 Kohlenstoffatomen oder eine Kombination dieser Reste, ist
- X: für die Gruppe 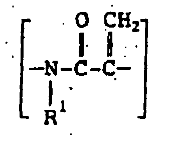 steht, die über das Stickstoffatom oder über C-2 an den Rest B gebunden ist, wobei die nicht mit B verbundene Bindungsstelle einen Rest R² trägt,
- R¹ =: Wasserstoff oder eine C₁- bis C₅-Alkylgruppe, insbesondere Wasserstoff oder eine C₁- bis C₃-Alkylgruppe,
- R² =: Wasserstoff oder eine C₁- bis C₅-Alkylgruppe, insbesondere Wasserstoff oder eine C₁- bis C₃-Alkylgruppe,
- n =: 2 oder 3, insbesondere 2.

Bei der Gruppe X handelt es sich um N-substituierte Amidgruppen, die über den Amidstickstoff oder über das Kohlenstoffatom C-2 an den Reste B gebunden sind. Diese beiden Bindungsvarianten können durch die Formeln (I) und (II) veranschaulicht werden. 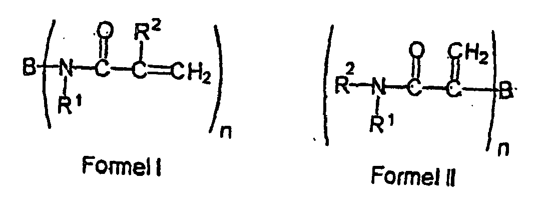 Amide der Formel I sind bevorzugt.

Erfindungsgemäß werden polyfunktionelle Amide BXₙ zur Herstellung von Dentalmaterialien verwendet, d.h. Amide, die mindestens zwei polymerisationsfähige Gruppen X, vorzugsweise mindestens zwei (Meth)acrylgruppen pro Molekül enthalten.

Zusätzlich zu den Resten X kann B einen oder mehrere, beispielsweise 1 bis 6 weitere Substituenten, insbesondere Cl, Br, ganz besonders bevorzugt OH und/oder COOH tragen. Vorzugsweise weist B 0 bis 3 weitere Substituenten auf.

Die Reste R¹ und R² können unabhängig voneinander ebenfalls einen oder mehrere, beispielsweise 1 bis 3 Substituenten tragen, die vorzugsweise aus Cl, Br, OH und/oder COOH ausgewählt sind. Vorzugsweise weisen R¹ und R² 0 bis 2 Substituenten auf.

Unter Kombinationen der Bedeutungen (1) bis (4) werden Gruppen verstanden, die sich beispielsweise aus mehreren alicyclischen oder aromatischen Resten zusammensetzen und die direkt oder über O, S, NH, CO-NH, NH-CO, NH-CO-O, O-CO-NH und/oder NH-CO-NH miteinander verbunden sind, z.B. -Ph-NH-Ph-, -Cyclohexylen-Cyclohexylen- und dergleichen.

Die Amide der Formel BXₙ sind radikalisch polymerisierbar und können allein oder vorzugsweise in Mischung mit anderen polymerisationsfähigen Komponenten in Gegenwart von geeigneten Polymerisationsinitiatoren, z.B. thermisch oder durch Einstrahlung von Licht des sichtbaren oder UV-Bereichs zu mechanisch stabilen Schichten, Form- oder Füllkörpern gehärtet werden.

Durch die Anzahl der polymerisationsfähigen Gruppen der multifunktionellen Amide BXₙ können die Vernetzungsdichte und damit die mechanischen Eigenschaften der ausgehärteten Materialien, wie E-Modul oder die Festigkeit, gezielt eingestellt werden. Darüber hinaus lassen sich durch die Art des organischen Grundgerüstes B, durch die ggf. weiter vorhandenen Substituenten, sowie durch die Art und Anzahl der Substituenten an den Amidgruppen die Löslichkeit, Funktionalität und Reaktivität der multifunktionellen Amide BXₙ variieren. Beispielsweise werden bei der Verwendung langkettiger Gruppen B relativ flexible Materialien erhalten, während kurzkettige Gruppen B zu eher steifen und harten Materialien führen. Mit der Anzahl der polymerisationsfähigen Gruppen steigt die Reaktivität der Monomeren und außerdem nimmt die erreichbare Vernetzungsdichte der ausgehärteten Materialien zu. Die Wasserlöslichkeit der Amide BXₙ kann durch OH- und/oder COOH-Substituenten erhöht werden. COOH-Gruppen können zudem durch Wechselwirkung mit Ca²⁺-Ionen die Haftung der Materialien an der Zahnhartsubstanz verbessern.

Die Amide BXₙ sind in Wasser, Alkohol, d.h. insbesondere Methanol und Ethanol, und in Mischungen dieser Lösungsmittel löslich und zeichnen sich vor allem durch eine hohe Hydrolysestabilität in Gegenwart von starken Säuren aus. Sie eignen sich besonders zur Herstellung dentaler Beschichtungsmaterialien, Zemente, Füllungsmaterialien und insbesondere Adhäsive.

Die bevorzugten erfindungsgemäßen multifunktionellen Amide der allgemeinen Formel I lassen sich durch Umsetzung von funktionellen (Meth)acrylsäure-Derivaten (R³ = Cl, O-CO-CR₂=CH₂, OAlkyl, OH) mit primären (R¹ = H) bzw. sekundären (R¹ = Alkyl oder Aryl)

Polyaminen unter Anwendung der aus der organischen Chemie bekannten Methoden für die Bildung von Amid-Bindungen herstellen (vgl. Methoden der Organischen Chemie, HOUBEN-WEYL Bd. E5 1985, Georg Thieme Verlag S. 941ff):

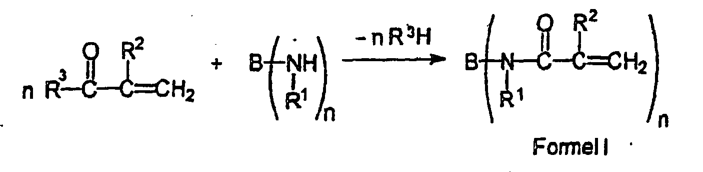

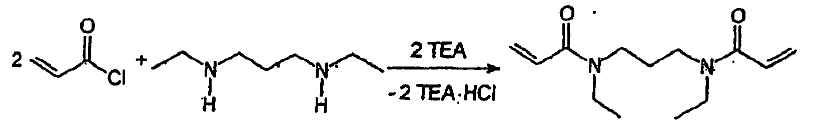

Dabei werden vorzugsweise die Säurechloride (R³ = C1) eingesetzt, die in Gegenwart einer äquimolarer Mengen an Hilfsbase, z.B. Triethylamin (TEA), mit dem Polyamin umgesetzt werden.

Konkretes Beispiel:

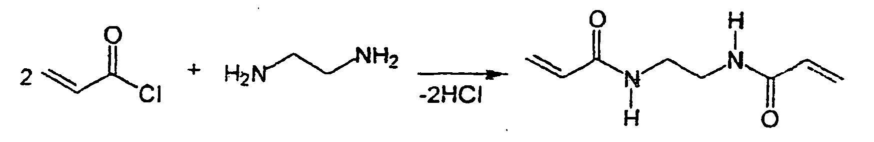

Vorteilhafterweise können die Polyamine auch selbst als Säurefänger eingesetzt werden (Überschuß), da die entsprechenden Hydrochloride sehr schwer löslich sind und sich somit leicht aus dem Reaktionsgemisch abtrennen lassen (J. A. Helpern, Synth. Comm. 10 (1980) 569).

Eine weitere Möglichkeit der Synthese von polyfunktionellen (Meth)acrylamiden besteht in der Umsetzung von N-(Hydroxyalkyl)-(meth)acrylamiden mit Polyisocyanaten, die zu einer Verknüpfung über hydrolysestabile Urethanbindungen führt. Die als Ausgangsverbindungen benötigten N-(Hydroxyalkyl)-(meth)acrylamide können aus reaktiven (Meth)acrylverbindungen (R³ = Cl, O-CO-CR₂=CH₂, OAlkyl) durch Umsetzung mit Aminoalkoholen erhalten werden (vgl. z.B. DE 3.412.650):

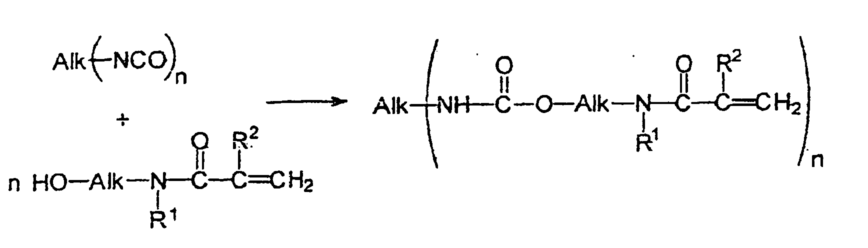

### Konkretes Beispiel:

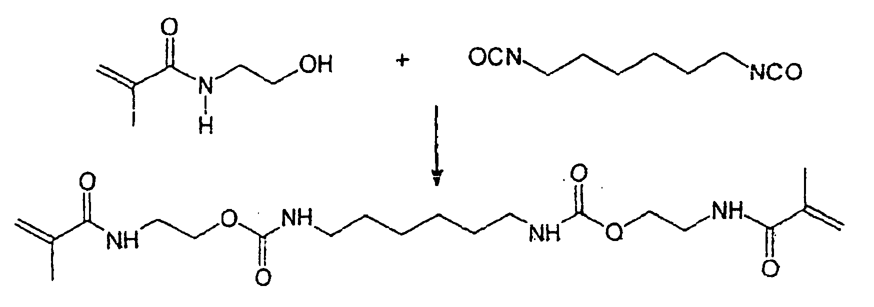

Die erfindungsgemäßen Amide der Formel II lassen sich z.B. durch Umsetzung von Acrylsäureestern mit Aldehyden erhalten (D. Basavaiah, P. D. Rao, R. S. Hyma, Tetrahedron 56 (1996) 8001). So führt die Reaktion von Acrylsäureestern mit Formaldehyd bzw. primären Aldehyden zu α-Hydroxymethylacrylaten, die mit polyfunktionellen elektrophilen Reagenzien zu den entsprechenden polyfunktionellen Methacrylaten umgesetzt werden können. Durch Verwendung von polyfunktionellen Aldehyden bzw. Formaldehyd bei Acrylatüberschuß können auch direkt polyfunktionelle (Meth)acrylsäureester erhalten werden. Der Austausch der Ester gegen die Amid-Funktion kann entweder durch Aminolyse oder durch Hydrolyse des Esters und nachfolgende Kondensation mit Aminen in Gegenwart eines Kondensationsmittels erfolgen.

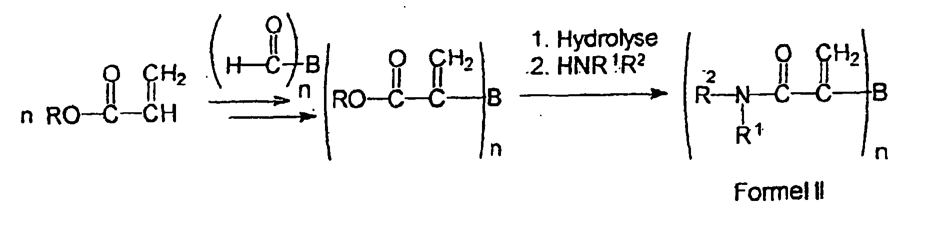

Als Kondensationsmittel für die Amidierung können Carbodiimide bzw. Phosphoroxychlorid (Houben-Weyl; Stuttgart 1974; Georg Thieme Verlag 4. Auflage Bd. 15/2 Peptide; S. 103ff und 232ff) eingesetzt werden.

### Konkretes Beispiel:

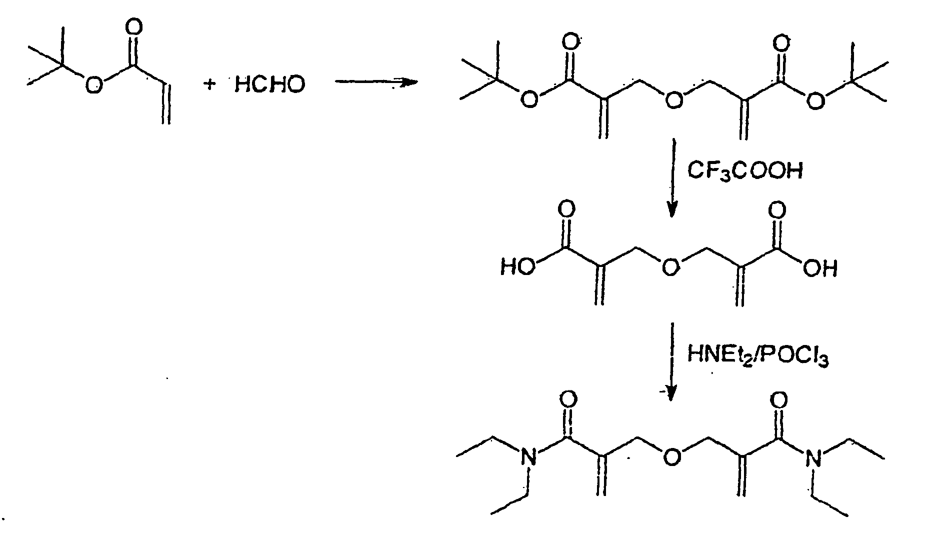

Konkrete Beispiele für die erfindungsgemäßen multifunktionellen (Meth)acrylamide der Formel (I) und (II) sind:

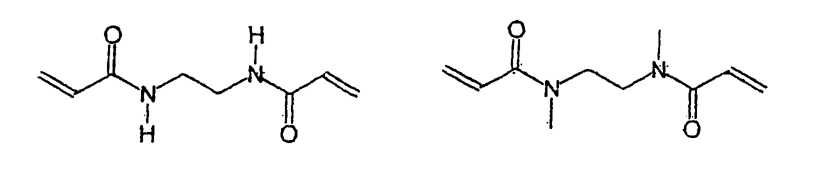

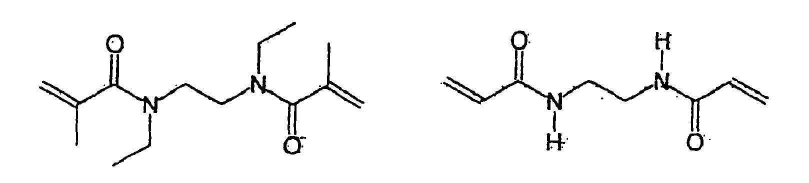

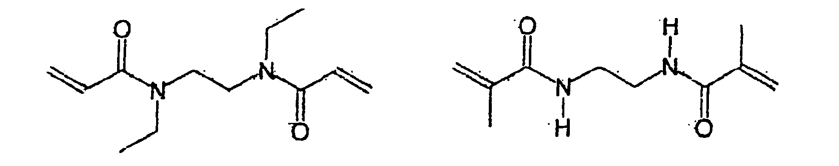

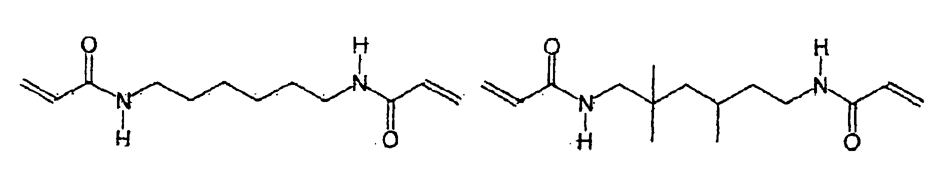

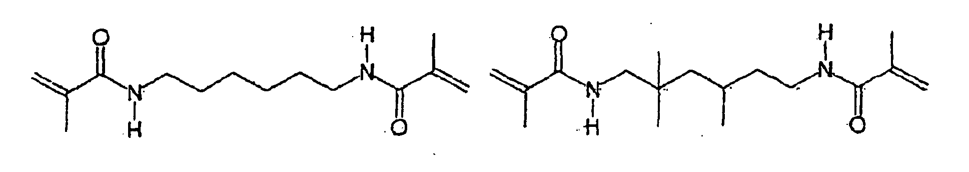

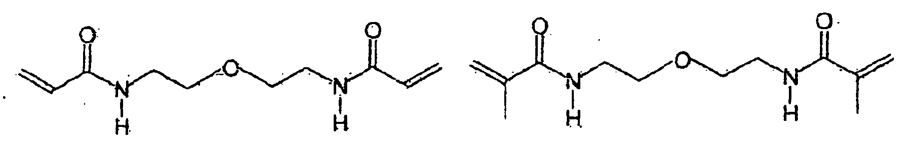

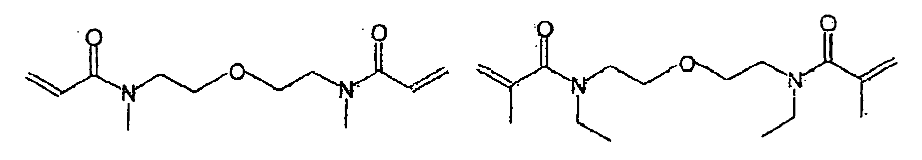

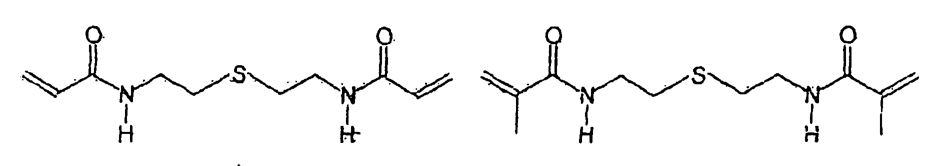

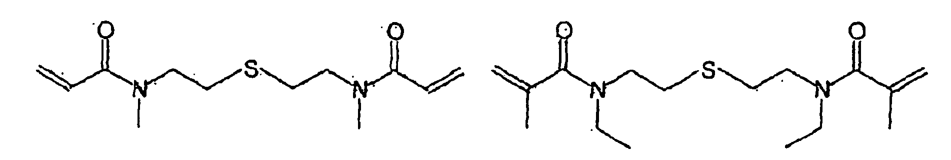

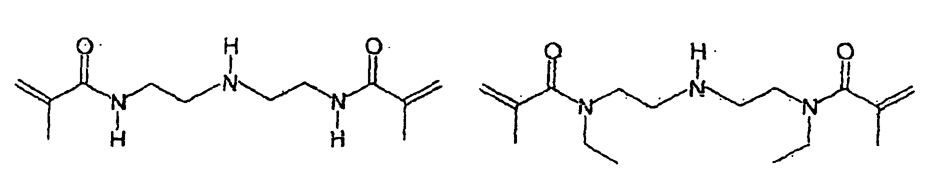

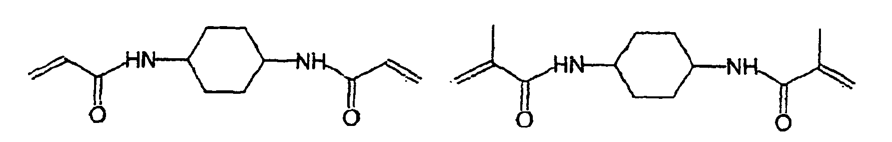

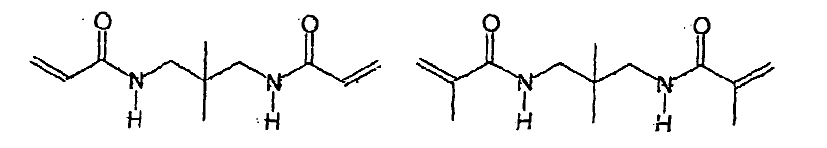

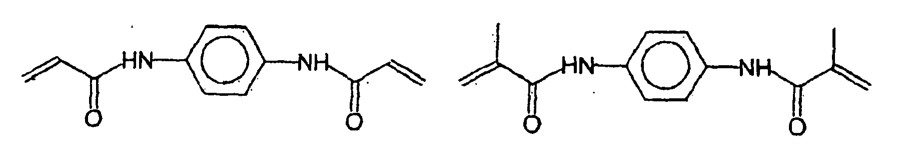

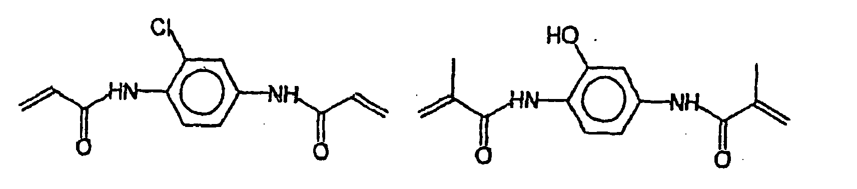

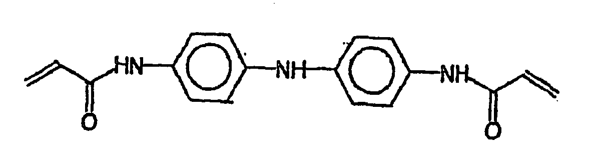

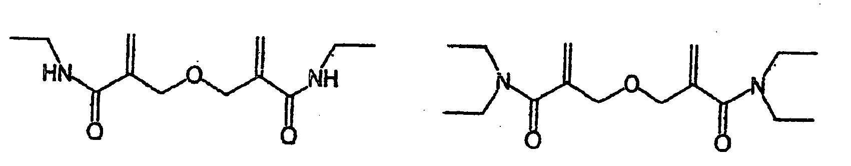

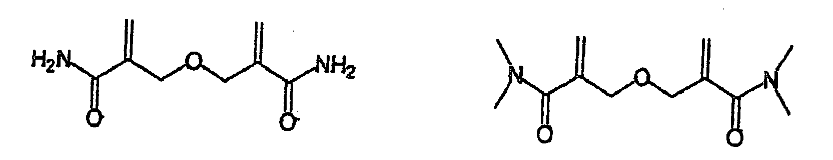

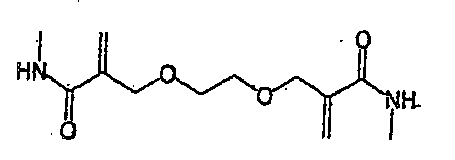

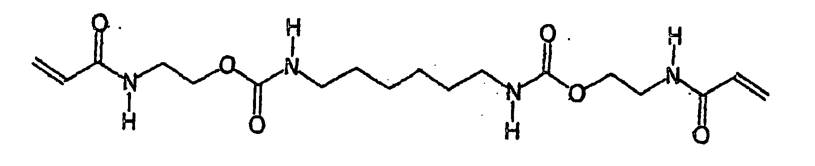

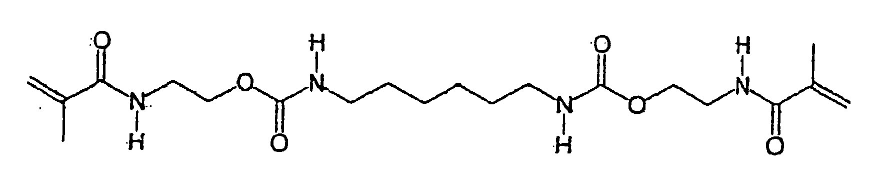

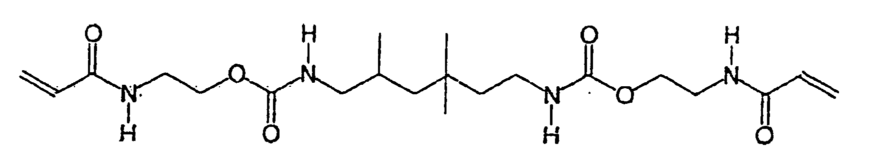

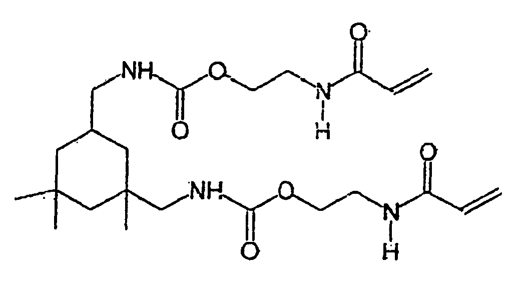

Zur Herstellung von Dentalmaterialien werden die multifunktionellen Amide BXₙ mit einem Polymerisationsinitiator, mindestens einem sauren polymerisierbaren Monomer und ggf. weiteren durch radikalische Polymerisation härtbare Bindemitteln, Lösungsmitteln, Füllstoffen und weiteren Additiven gemischt.

Die genaue Zusammensetzung der Materialien hängt vom jeweiligen Verwendungszweck ab. Adhäsive enthalten neben mindestens einem Amid der Formel BXₙ vorzugsweise Polymerisationsinitiator, mindestens ein saures polymerisierbares Monomer und Lösungsmittel.

Zemente enthalten neben mindestens einem Amid der Formel BXₙ vorzugsweise Polymerisationsinitiator, mindestens ein saures polymerisierbares Monomer und Füllstoff.

Beschichtungsmaterialien enthalten neben mindestens einem Amid der Formel BXₙ vorzugsweise Polymerisationsinitiator und Lösungsmittel und Füllungsmaterialien mindestens ein Amid der Formel BXₙ, Polymerisationsinitiator und Füllstoff.

In jedem Fall beträgt die Menge des oder der Amide BXₙ bezogen auf die Gesamtmasse des Dentalmaterials vorzugsweise mindestens 1 Gew.-%, insbesondere mindestens 5 Gew.-%.

Als organisches Bindemittel eignen sich alle durch Polymerisation härtbaren Bindemittel, insbesondere ethylenisch ungesättigte, polymerisierbare Monomere (acide und nicht-acide), wie monofunktionelle oder polyfunktionelle (Meth)acrylate und (Meth)acrylamide, die allein oder in Mischungen eingesetzt werden können. Monofunktionelle Monomere enthalten eine, polyfunktionelle Monomere zwei polymerisationsfähige Gruppen. Das Bindemittel enthält vorzugsweise keine Amine mit zwei oder mehr Aminogruppen.

Als bevorzugte radikalisch polymerisationsfähige monofunktionelle Monomere kommen Mono(meth)acrylamide und Mono(meth)acrylate, z.B. Acrylamid, Methacrylamid, N-Ethylacrylamid, Methyl-, Ethyl-, Butyl-, Benzyl-, Furfuryl- oder Phenyl(meth)acrylat in Frage.

Bevorzugte polyfunktionelle Monomere sind die bekannten polyfunktionellen Acrylate bzw. Methacrylate, wie z.B. Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Hexamethylendiisocyanat), Di-, Tri- oder Tetraethylenglycoldi(meth)acrylat, Decandiol-di(meth)acrylat, Trimethylolpropantri(meth)acrylat, Pentaery-thrittetra(meth)acrylat sowie Butandioldi(meth)acrylat, 1,10-Decandioldi(meth)acrylat oder 1,12-Dodecandioldi(meth)acrylat.

Die multifunktionellen Amide BXₙ eigenen sich besonders als Vernetzer für stark saure polymerisationsfähige Monomere, die häufig in Adhäsiven zur Anwendung kommen. Dazu gehören insbesondere Phosphorsäureestermethacrylate, wie z.B. 2-(Methacryloyloxyethyl)-dihydrogenphosphat, Di-(2-methacryloyloxyethylhydrogenphosphat oder Dipentaerythritolpentamethacryloyloxydihydrogenphosphat (vgl. N. Nakabayashi, P. D. Pashley, Hybridization of dental hard tissues, Quintess. Publ. Tokyo etc. 1998, 9 ff). Besonders bevorzugt sind hydrolysestabile Acrylphosphonsäuren, wie z. B. 2-[3-(Dihydroxyphosphoryl)-oxa-propyl]acrylsäureethylester oder 1,2-Bis[1-dihydroxyphosphoryl)-1-[2-methylen-3-ylpropansäureethylester)oxy]methyl]-benzol, die in der DE 197.46.708 C2 beschrieben werden. Zur Unterscheidung zwischen aciden und nicht-aciden Monomeren wird der Begriff "polymerisierbares Monomer" nur für nicht-acide Komponenten verwendet, der Begriff "polymerisierbares Bindemittel" umfaßt acide und nicht-acide Substanzen.

Bei der Verwendung weiterer polymerisierbarer Monomore (acide und nicht-acide), beträgt die Menge des oder der Amide BXₙ bezogen auf die Summe der Massen des oder der Amide BXₙ und der polymerisierbaren Monomere vorzugsweise mehr als 3 Gew.-%, besonders bevorzugt mehr als 10 Gew.-%.

Die Aushärtung der Zusammensetzungen kann je nach Art des verwendeten Polymerisationsinitiators durch thermische, photochemische oder redoxinduzierte radikalische Polymerisation erfolgen. Bevorzugte Beispiele für thermische Initiatoren sind die bekannten Peroxide, wie z.B. Dibenzoylperoxid, Dilaurylperoxid, tert.-Butylperoctoat oder tert.-Butylperbenzoat sowie Azobisisobutyroethylester, Azobisisobutyronitril, Azobis-(2-methylpropionami-din)dihydrochlorid Benzpinakol oder 2,2-Dimethylbenzpinakol.

Bevorzugte Photoinitiatoren sind Benzophenon, Benzoin sowie deren Derivate oder α-Diketone oder deren Derivate wie 9,10-Phenanthrenchinon, Diacetyl oder 4,4-Dichlorbenzil. Besonders bevorzugt werden Campherchinon und 2,2-Dimethoxy-2-phenylacetophenon und besonders bevorzugt α-Diketone in Kombination mit Aminen als Reduktionsmittel, wie z.B. 4-(N,N-Dimethylamino)-benzoesäureester, N,N-Dimethylaminoethylmethacrylat, N,N-Dimethyl-sym.-xylidin oder Triethanolamin, eingesetzt. Darüber hinaus sind auch Acylphosphine, wie z.B. 2,4,6-Trimethylbenzoyldiphenyl- oder Bis(2,6-dichlorbenzoyl)-4-N-propylphenylphosphinoxid besonders geeignet.

Als Initiatoren für die bei Raumtemperatur durchgeführte Polymerisation werden vorzugsweise Redox-Initiatorkombinationen, wie z.B. Kombinationen von Benzoyl- oder Laurylperoxid mit N,N-Dimethyl-sym.-xylidin oder N,N-Dimethyl-p-toluidin, verwendet.

Zusammensetzungen, die einen Initiator für die Photopolymerisation enthalten, sind bevorzugt.

Als Lösungsmittel für die multifunktionellen Amide BXₙ können vor allem polare Lösungsmittel, wie Wasser, Ethanol, Aceton, Acetonitril oder Mischungen aus diesen Lösungsmitteln eingesetzt werden.

Weiterhin können die erfindungsgemäß eingesetzten Zusammensetzungen zur Verbesserung der mechanischen Eigenschaften mit anorganischen Partikeln oder Fasern gefüllt werden. Bevorzugte anorganische partikuläre Füllstoffe sind amorphe kugelförmige Materialien auf der Basis von Oxiden, wie ZrO₂ und TiO₂ bzw. Mischoxiden aus SiO₂, ZrO₂ und/oder TiO₂ mit einer mittleren durchschnittlichen Partikelgröße von 0,005 bis 2,0 µm, vorzugsweise von 0,1 bis 1 µm, wie sie beispielsweise in der DE-PS 32 47 800 offenbart werden, nanopartikuläre oder mikrofeine Füllstoffe, wie pyrogene Kieselsäure oder Fällungskieselsäure sowie Makro- oder Minifüllstoffe, wie Quarz-, Glaskeramik- oder Glaspulver mit einer durchschnittlichen Teilchengröße von 0,01 bis 20 µm, vorzugsweise 0,01 bis 5 µm sowie röntgenopake Füllstoffe, wie Ytterbiumtrifluorid. Unter Mini-Füllstoffen werden Füllstoffe mit einer Partikelgröße von 0,5 bis 1,5 µm und unter Makro-Füllstoffen Füllstoffe mit einer Partikelgröße von 10 bis 20 µm verstanden.

Gegebenenfalls können die erfindungsgemäß eingesetzten Zusammensetzungen weitere Additive enthalten, wie z.B. Farbmittel (Pigmente oder Farbstoffe), Stabilisatoren, Aromastoffe, mikrobizide Wirkstoffe, Weichmacher oder UV-Absorber.

Ein besonders bevorzugtes Dentalmaterial enthält, jeweils bezogen auf die Gesamtmasse des Dentalmaterials:
(a) 1 bis 90 Gew.-%, insbesondere 5 bis 50 Gew.-%, ganz besonders bevorzugt 8 bis 40 Gew-% Amid der Formel BXₙ,
(b) 0,1 bis 5,0 Gew.-%, insbesondere 0,2 bis 2,0 Gew.-% Polymerisationsinitiator,
(c) 0 bis 70 Gew.-%, insbesondere 0 bis 50 Gew.-% polymerisierbares Monomer,
(d) > 0 bis 70 Gew.-, vorzugsweise 0 bis 50 Gew.-%, besonders bevorzugt 0 bis 40 Gew.-% acides Monomer,
(e) 0 bis 70 Gew.-%, insbesondere 0 bis 50 Gew.-%, Füllstoff, und/oder
(f) 0 bis 70 Gew.-%, insbesondere 0 bis 50 Gew.-% Lösungsmittel.

Zusammensetzungen, die als Komponente (c) mindestens ein polyfunktionelles polymerisierbares Monomer enthalten, sind bevorzugt, wobei der Anteil des oder der polyfunktionellen Monomere an der Komponente (c) gemäß einer besonders bevorzugten Ausführungform mindestens 50 Gew.-% bezogen auf die Masse der Komponente (c) beträgt.

Diese Zusammensetzung kann für bestimmte Anwendungszwecke weiter optimiert werden. So enthält ein Dentalmaterial, das sich besonders als Adhäsiv eignet, jeweils bezogen auf die Gesamtmasse des Dentalmaterials vorzugsweise:
(a) 5 bis 40 Gew.-% Amid der Formel BXₙ,
(b) 0,2 bis 2,0 Gew.-% Polymerisationsinitiator,
(c) 0 bis 40 Gew.-% polymerisierbares Monomer,
(d) 5 bis 40 Gew.- acides Monomer und
(f) 2 bis 50 Gew.-% Lösungsmittel.

Ein Dentalmaterial, das sich besonders als dentaler Zement eignet, enthält, jeweils bezogen auf die Gesamtmasse des Dentalmaterials vorzugsweise:
(a) 5 bis 20 Gew.-% Amid der Formel BXₙ,
(b) 0,2 bis 2,0 Gew.-% Polymerisationsinitiator,
(c) 0 bis 20 Gew.-% polymerisierbares Monomer,
(d) 2 bis 20 Gew.-% acides Monomer und
(e) 5 bis 60 Gew.-% Füllstoff.

Ein Dentalmaterial, das sich besonders als dentales Beschichtungsmaterial eignet, enthält, jeweils bezogen auf die Gesamtmasse des Dentalmaterials vorzugsweise:
(a) 5 bis 40 Gew.-% Amid der Formel BXₙ,
(b) 0,2 bis 2,0 Gew.-% Polymerisationsinitiator,
(c) 0 bis 50 Gew.-% polymerisierbares Monomer und
(f) 5 bis 50 Gew.-% Lösungsmittel.

Ein Dentalmaterial, das sich besonders als dentales Füllungsmaterial eignet, enthält, jeweils bezogen auf die Gesamtmasse des Dentalmaterials vorzugsweise:
(a) 5 bis 20 Gew.-%, Amid der Formel BXₙ,
(b) 0,2 bis 2,0 Gew.-% Polymerisationsinitiator,
(c) 0 bis 30 Gew.-% polymerisierbares Monomer und
(e) 10 bis 70 Gew.-% Füllstoff.

Die erfindungsgemäßen Dentalmaterialien zeichnen sich im gehärteten und ungehärteten Zustand durch eine hohe Hydrolysestabilität insbesondere auch in Gegenwart von aciden Verbindungen aus und sind damit auch im ungehärteten Zustand lagerstabil.

Die Erfindung wird im folgenden anhand von Beispielen näher erläutert. Wenn nicht anders angegeben, sind alle Prozentangaben Gew.-%.

### Beispiel 1

**Synthese von Ethylenbisacrylamid (1) (**J. A. Helpern, Synth. Comm. 10 (1980) 569**)**

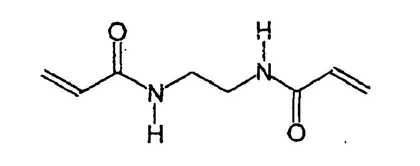

In einem 6-1-Sulfierkolben wurden 90,5 g (1,0 mol) Acrylsäurechlorid und 16 mg Hydrochinonmonomethylether (MEHQ, Stabilisator) in 3 l Methylenchlorid gelöst und auf 0 °C abgekühlt. Dazu wurde unter Rühren eine Lösung von 60,1 g (1,0 mol) Ethylendiamin in 2 l Methylenchlorid so zugetropft, daß die Temperatur zwischen 0-5 °C blieb. Nach 6 h Rühren wurde die Mischung auf Raumtemperatur erwärmen gelassen, der gebildete Niederschlag abfiltriert und der Feststoff mit 1 l Acetonitril gewaschen. Das Filtrat und die Waschlösung wurden vereinigt und im Vakuum zu einer dicken Suspension eingeengt. Danach wurde der Feststoff abfiltriert, getrocknet und aus ca. 1,3 l Aceton umkristallisiert. Es wurden 42,5 g (56 % Ausbeute) eines weißen Feststoffes mit einem Schmelzpunkt von 142-145 °C erhalten.

¹H-NMR (400 MHz, CDCl₃): δ = 3,15-3,30 (m; 4H, CH₂CH₂), 5,55-5,65 und 6,02-6,28 (m; 2H+4H, CH=CH₂) ppm.

### Beispiel 2

### Synthese von N,N'-Diethyl-1,3-propylen-bis-acrylamid (2)

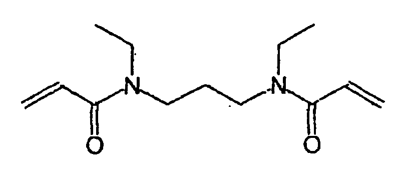

In einem 2,5-l-Sulfierkolben wurden 36,3 g (0,40 mol) Acrylsäurechlorid und 4 mg MEHQ in 1,2 l Acetonitril gelöst und auf -5 °C abgekühlt. Dazu wurde unter Rühren eine Lösung von 46,9 g (0,36 mol) N,N'-Diethylpropylendiamin in 1,2 l Acetonitril so zugetropft, daß die Temperatur zwischen -5 und 0°C blieb. Nach 1,5 h wurde die Mischung auf Raumtemperatur erwärmen gelassen und für weitere 4 h gerührt, der gebildete Niederschlag anschließend abfiltriert und mit 0,5 l Acetonitril gewaschen. Die Acetonitrilphasen wurden vereinigt und im Vakuum (10 mbar, 40 °C) eingeengt. Das Rohprodukt wurde in 150 ml Aceton aufgenommen, über eine Fritte mit 50 g Kieselgel 60 filtriert und erneut eingeengt. Nach Wiederholung dieser Prozedur blieben 32,7 g (76 % Ausbeute ) einer schwach gelblichen Flüssigkeit (η (23 °C) = 270 mPa·s) zurück.
¹H-NMR (400 MHz, DMSO-d₆): δ = 1,10-1,25 (m; 6H, CH₃), 1,80-1,92 (m; 2H, CH₂CH,-CH₂), 3,35-3,61 (m; 8H, CH₂N), 5,63-5,77, 6,28-6,42 und 6,47-6,66 (m; 3x2H, CH=CH₂) ppm.

### Beispiel 3

### Synthese von Bis[2-(2-methyl-acrylamino)-ethoxycarbonyl]-hexamethylendiamin (3)

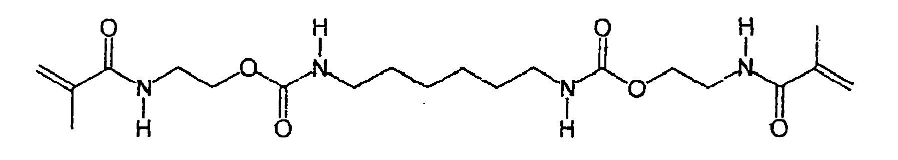

6,44 g (49,9 mmol) N-(2-Hydroxyethyl)-methacrylamid und 12 mg MEHQ wurden in 25 ml wasserfreiem Methylenchlorid gelöst. Nach Zugabe von 2 Tropfen Dibutylzinndioctanoat als Katalysator (Metatin 812) wurden unter Kühlung 4,19 g (24,9 mmol) Hexamethylendiisocyanat so zugetropft, daß die Temperatur bei ca. 5 °C blieb. Dann wurde für ca. 4 d bei Raumtemperatur gerührt, das Lösungsmittel entfernt und das feste Rohprodukt zweimal aus wasserfreiem Ethanol umkristallisiert. Es ergaben sich 4,8 g (45 % Ausbeute) eines farblosen kristallinen Feststoffs (Schmp.: 154-155 °C unter Polymerisation).

¹H NMR (400 MHz, DMSO-d₆): δ = 1,22 ( br. s; 4 H, CH₂CH₂CH₂CH₂CH₂CH₂), 1,35 (br. s, 4H, CH₂CH₂CH₂CH₂CH₂CH₂), 1,84 (s; 6H, CH₃), 2.91-2.96, (m; 4H, CH₂NHCOO), 3,28-3,34 (m; 4H, CH₂NHCOC=C), 3,92-4,00 (m; 4H, CH₂O), 5,32, 5,66 (s; 4H, CH₂=), 6,8, 7,11 und 7,98 (br.; 0.25H + 1.75H + 2H, NH, H/D-Austausch).

### Beispiel 4

### Synthese von N,N'-(Dimethyl)-ethylen-bis-acrylamid (4)

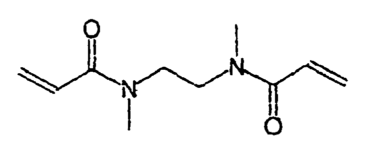

In einem 1,5-1-Sulfierkolben wurden 36,2 g (0,40 mol) Acrylsäurechlorid und 16 mg MEHQ in 600 ml l Methylenchlorid gelöst und auf -5 °C abgekühlt. Dazu wurde unter Rühren eine Mischung aus 17,6 g (0,20 mol) N,N'-Dimethylethylendiamin, 40,8 g (0,40 mol) Triethylamin und 400 ml Methylenchlorid so zugetropft, daß die Temperatur zwischen -5 und 0 °C blieb. Nach 1,5 h Rühren wurde die Mischung auf Raumtemperatur erwärmen gelassen, über Nacht gerührt, der gebildete Niederschlag abfiltriert und das Filtrat im Vakuum eingeengt. Das Rohprodukt wurde in 150 ml Aceton aufgenommen, über eine Fritte mit 50 g Kieselgel 60 filtriert und erneut eingeengt. Nach Wiederholung dieser Prozedur blieben 30,1 g (77 % Ausbeute ) einer schwach gelblichen Flüssigkeit zurück.
¹H-NMR (400 MHz, CDCl₃): δ = 3,10 und 3,14 (s; 2x3H, CH₃), 3,54-3,67 (2m; 4H, CH₂N), 5,68, 6,35 und 6,56 (m; 3x2H, CH=CH₂) ppm.

### Beispiel 5

### Synthese von 2,2,4-Trimethylhexamethylen-1,6-bismethacrylamid (5)

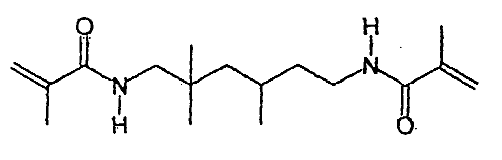

Unter Eiskühlung wurden 142,0 g (0,90 mol) 2,2,4-Trimethylhexamethylendiamin in 2 l Acetonitril zu einer Lösung von 104.5 g (1,0 mol) Methacrylsäurechlorid und 20 mg Phenothiazin in 3 l Acetonitril getropft. Nach 16 h Rühren bei Raumtemperatur wurde die entstandene weiße Suspension abfiltriert. Das Filtrat wurde am Rotationsverdampfer unter Einleiten trockener Luft eingeengt. Das verbleibende Produkt wurde in 1 l Methylenchlorid gelöst und mehrfach mit je 1 l 2 N HCl gewaschen, wobei sich mittels Dünnschichtchromatographie (DC) kein Diamin mehr nachweisen ließ. Nach dem weiteren Auswaschen mit 500 ml 2 N Natronlauge und 500 ml Salzwasser wurde die Methylenchlorid-Lösung über Natriumsulfat getrocknet und das Lösungsmittel am Rotationsverdampfer bei 40 °C vollständig abdestilliert. Es verblieben 124,5 g (94 % Ausbeute) einer klaren viskosen Flüssigkeit.
¹H-NMR (400 MHz, CDCl₃): δ = 0,88-0,97 (m; 9H, (CH₃)₂C+CH₃-CH), 1,46-1,69 (m; 5H, CH₂CHCH₂), 1,95 (s; 6H, =C-CH₃), 3,25-3,32 (m; 4H, CH₂N), 5,32 und 5,69 (s; 2x2H, =CH₂) und 6,50-6,71 (br; 2H, NH) ppm.

### Beispiel 6

### Synthese von 3,3'-Oxybis(2-methylenpropionsäurediethylamid) (6)

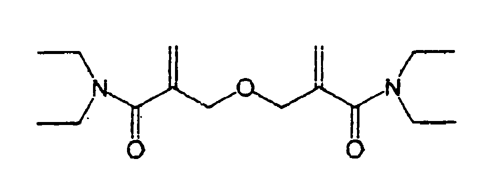

In einem 2,5-l-Sulfierkolben wurden 37,2 g (0,20 mol) 3,3'-Oxybis(2-methylenpropionsäure) in 1,75 l Tetrahydrofuran (THF) gelöst und auf -20 °C abgekühlt. Dazu wurden unter Rühren 29,2 g (0,40 mol) Diethylamin so zugetropft, daß die Temperatur -15 °C nicht überstieg. Anschließend wurden bei -15 °C erst 61,2 g (0,40 mol) Phosphorylchlorid und dann 121,2 g (1,2 mol) Triethylamin zugetropft und die Mischung für weitere 6 h gerührt. Nach Stehen über Nacht bei Raumtemperatur wurde der gebildete Niederschlag abfiltriert und mit 250 ml THF gewaschen. Die vereinigten THF-Phasen wurden im Vakuum eingeengt und mit 200 ml Wasser versetzt. Die entstehende klare Lösung wurde mehrfach mit je 100 ml Methylenchlorid extrahiert. Anschließend wurde das Extrakt mit 2N NaOH gewaschen, über wasserfreiem Natriumsulfat getrocknet und eingedampft. Das flüssige Rohprodukt wurde im Feinvakuum destilliert, wobei 18,5 g (31 % Ausbeute) eines hellgelben Öls (η (23 °C) = 170 mPa·s) bei 148 °C (0,04 mbar) isoliert wurden.
1H-NMR (400 MHz, CDCl₃): δ = 1,16 (t; 12H, CH₃), 3,42 (q; 8H, CH₂N), 4,23 (s; 4H, OCH₂=), 5,19 und 5,38 (m; 2x2H, =CH₂) ppm.

### Beispiel 7

### Radikalische Polymerisation der Amide 1 bis 5

In Schlenkgefäßen wurden homogene Mischungen aus 30 % jeweils eines der Amide der Beispiele 1 bis 5 bzw. Glycerindimethacrylat als Vergleichsbeispiel, 20 % Wasser, 49 % Ethanol und 1 % 2,2'-Azobis-(2-methylpropionamidin)-dihydrochlorid (Initiator) hergestellt und mittels Durchleiten von Argon entlüftet. Danach wurden die Polymerisationsansätze in einem Thermostat auf 65 °C erwärmt. Die Zeit, die verstrich bis sich ein dreidimensionales standfestes Gel gebildet hatte, wurde bestimmt (Gelzeit).

| **Monomer** | **Gelzeit [Minuten]** | **Hydrolysestabilität^{a)}** |
|---|---|---|
| Beispiel 1 | 1 | + |
| Beispiel 2 | 4 | + |
| Beispiel 3 | 6 | + |
| Beispiel 4 | 9 | + |
| Beispiel 5 | 7 | + |
| Glycerindimethacrylat (Vergleichsbeipiel) | 4 | - |

| | | |
|---|---|---|
| a) Zur Ermittlung der Hydrolysestabilität wurde eine 20 Gew.-%-ige Lösung des betreffenden Monomers in einer 1:1:1 Mischung aus Wasser, Ethanol und 10 %-iger Phosphorsäure bei 37°C gelagert. Nach 4 Wochen wurde die Lösung ¹H-NMR-spektroskopische untersucht. +: es wurde keine Hydrolyse des Monomers beobachtet -: das Polymer wurde vollständig abgebaut | | |

Die Polymerisationsversuche zeigen, daß die Amide BX₂ eine variierbare Polymerisationsfähigkeit besitzen. Das Bisacrylamid aus Beispiel 1 ist deutlich reaktiver, das Amid aus Beispiel 2 gleich reaktiv und das Bismethacrylamid z.B. aus Beispiel 4 weniger reaktiv als Glycerindimethacrylat. Als Maß für die Polymerisationsfähigkeit dient die Gelzeit. Dies ist die Zeit, die vergeht, bis sich aus einer Vernetzer und Monomer enthaltenden Lösung ein dreidimensionales Polymernetzwerk, d.h. ein sogenanntes Gel, gebildet hat. Je kürzer die Gelzeit ist, um so reaktiver ist das entsprechende Vernetzermonomer. Als Vergleichsverbindung wurde Glycerindimethacrylat gewählt, da es das einzige herkömmliche Vernetzermonomer ist, das sowohl eine gewisse Wasserlöslichkeit als auch eine sehr gute radikalische Polymerisationsfähigkeit aufweist.

Ein wesentlicher Vorteil der erfindungsgemäßen Amide ist in ihrer Hydrolysestabilität zu sehen. Wäßrige Lösungen der Amide sind damit auch in Gegenwart von aciden Verbindungen lagerstabil. Da bei der Polymerisation eine Verknüpfung der Monomerbausteine über kovalente C-C-Einfachbidungen erfolgt, gilt dies auch für die entsprechenden Polymerisate. Demgegenüber wird Glycerindimethacrylat in Gegenwart von Phosphorsäure vollständig zu Glycerin und Methacrylsäure gespalten.

### Beispiel 8

### N,N'-(Diethyl)-1,3-propylen-bis-acrylamid enthaltendes Dentinadhäsiv (2)

Zur Untersuchung der Dentinhaftung auf Rinderzahndentin wurde ein Adhäsiv folgender Zusammensetzung (Angabe in Gew.-%) hergestellt:

| | |
|---|---|
| Stark saures Adhäsivmonomer^{a)}: | 11,1 % |
| Glycerindimethacrylat: | 11,0 % |
| 2-Hydroxyethylmethacrylat: | 20,0 % |
| Ethanol: | 24,0 % |
| Bisacrylamid 2: | 33,1 % |
| Photoinitiator: | 0,8 % |

| | |
|---|---|
| ^{a)} 2-[3-(Dihydroxyphosphoryl)-2-oxa-butyl]-acrylsäureethylester (vgl. DE 197 46 708 C2) | |

Rinderzähne wurden so in einen Kunststoffzylinder eingebettet, daß sich das Dentin und der Kunststoff in einer Ebene befanden.
Die freie Fläche des Zahns wurde mit 37%-iger Phosphorsäure behandelt und nach 15 s Ätzung gründlich mit Wasser abgespült.
Durch die Säureätzung wurden die Dentintubili geöffnet. Dann wurde mit einem kleinen Pinsel eine Schicht Adhäsiv obiger Zusammensetzung aufgebracht, die behandelte Fläche zur Entfernung des Lösungsmittels kurz mit einem Luftstrahl angeblasen und für 40 s mit einer Halogenlampe mit Licht einer Wellenlänge von 390 bis 500 nm (Astralis 7, Vivadent) belichtet. Auf die gehärtete Adhäsivschicht wurde ein Kompositzylinder aus Tetric® Ceram (Vivadent, 16 Gew.-% polymerisierbares Monomer, 0,5 Gew.-% Photoinitiator und Stabilisator, 83,5 Gew.-% Füllstoff) in zwei Schichten von je 1-2 mm polymerisiert. Anschließend wurden die so präparierten Zähne für 24 h bei 37 °C in Wasser gelagert und die Scherhaftfestigkeit bestimmt. Es wurde ein Wert von 15,4 MPa gemessen.

## Patentansprüche

1. Dentalmaterial enthaltend ein Amid der allgemeinen Formel BXₙ in der
B für einen n-fach mit der Gruppe X substituierten Kohlenwasserstoffrest mit 2 bis 50 Kohlenstoffatomen steht, der eine oder mehrere der Gruppen O, S, NH, CO-NH, O-CO-NH und/oder NH-CO-NH enthalten kann, wobei
B eine gesättigte, lineare oder verzweigte aliphatische Gruppe mit 2 bis 15 Kohlenstoffatomen, die eine oder zwei der Gruppen S, NH, O, NH-CO-O oder O-CO-NH enthalten kann,
eine cycloaliphatische Gruppe mit 6 oder 15 Kohlenstoffatomen,
eine aromatischer oder nicht-aromatischer heterocyclischer Rest mit 3 bis 10 Kohlenstoffatomen und 1 bis 3 Heteroatomen,
eine aromatischer Rest mit 6 bis 12 Kohlenstoffatomen oder
eine Kombination dieser Reste ist,
X für die Gruppe 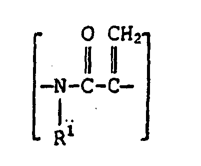 steht, die über das Stickstoffatom oder über C-2 an den Rest B gebunden ist, wobei die nicht mit B verbundene Bindungsstelle einen Rest R² trägt,
R¹ Wasserstoff oder eine C₁- bis C₅-Alkylgruppe ist,
R² Wasserstoff oder eine C₁- bis C₅-Alkylgruppe ist,
n 2 oder 3 ist,
mindestens ein saures polymerisierbares Monomer, und einen Polymerisationsinitiator.

2. Dentalmaterial nach Anspruch 1, **dadurch gekennzeichnet, daß** B zusätzlich zur der Gruppe X einen oder mehrere Substituenten trägt, die aus Cl, Br, OH und/oder COOH ausgewählt sind.

3. Dentalmaterial nach einem der Ansprüche 1 bis 2, **dadurch gekennzeichnet, daß** R¹ und/oder R² ein oder mehrfach substituiert sind, wobei der oder die Substituenten aus Cl, Br, OH und/oder COOH ausgewählt sind.

4. Dentalmaterial nach einem der Ansprüche 1 bis 3, **dadurch gekennzeichnet, daß** es ein polymerisierbares Bindemittel enthält.

5. Dentalmaterial nach Anspruch 3 oder 4, **dadurch gekennzeichnet, daß** es mindestens ein ethylenisch ungesättigtes polymerisierbares Monomer enthält.

6. Dentalmaterial nach Anspruch 5, **dadurch gekennzeichnet, daß** es ein polyfunktionelles polymerisierbares Monomer enthält.

7. Dentalmaterial nach einem der Ansprüche 3 bis 6, **dadurch gekennzeichnet, daß** die Menge des Amids BXₙ bezogen auf die Summe der Massen des Amids BXₙ und sonstiger polymerisierbarer Monomere mehr als 3 Gew.-%, vorzugsweise mehr als 10 Gew.-% beträgt.

8. Dentalmaterial nach einem der Ansprüche 3 bis 7, **dadurch gekennzeichnet, daß** es einen Initiator für die Photopolymerisation enthält.

9. Dentalmaterial nach einem der Ansprüche 1 bis 8, **dadurch gekennzeichnet, daß** es Füllstoff enthält.

10. Dentalmaterial nach einem der Ansprüche 1 bis 9, **dadurch gekennzeichnet, daß** es bezogen auf die Gesamtmasse des Dentalmaterials mindestens 1 Gew.-% vorzugsweise mindestens 5 Gew.-% des Amids BXₙ enthält.

11. Dentalmaterial nach einem der Ansprüche 1 bis 4, **dadurch gekennzeichnet, daß** es
(a) 1 bis 90 Gew.-% des Amids BXₙ,
(b) 0,1 bis 5,0 Gew.-% Polymerisationsinitiator,
(c) 0 bis 70 Gew.-% polymerisierbares Monomer (nicht-acide),
(d) > 0 bis 70 Gew.-% acides polymerisierbares Monomer,
(e) 0 bis 70 Gew.-% Füllstoff,
(f) 0 bis 70 Gew.-% Lösungsmittel
enthält, jeweils bezogen auf die Gesamtmasse des Dentalmaterials.

12. Verwendung eines Amids der allgemeinen Formel BXₙ in der
B für einen n-fach mit der Gruppe X substituierten Kohlenwasserstoffrest mit 2 bis 50 Kohlenstoffatomen steht, der eine oder mehrere der Gruppen O, S, NH, CO-NH, O-CO-NH und/oder NH-CO-NH enthalten kann, wobei
B eine gesättigte, lineare oder verzweigte aliphatische Gruppe mit 2 bis 15 Kohlenstoffatomen, die eine oder zwei der Gruppen S, NH, O, NH-CO-O oder O-CO-NH enthalten kann,
eine cycloaliphatische Gruppe mit 6 oder 15 Kohlenstoffatomen,
eine aromatischer oder nicht-aromatischer heterocyclischer Rest mit 3 bis 10 Kohlenstoffatomen und 1 bis 3 Heteroatomen,
eine aromatischer Rest mit 6 bis 12 Kohlenstoffatomen oder
eine Kombination dieser Reste ist,
X für die Gruppe 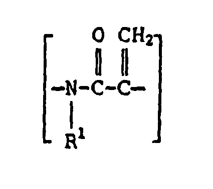 steht, die über das Stickstoffatom oder über C-2 an den Rest B gebunden ist, wobei die nicht mit B verbundene Bindungsstelle einen Rest R² trägt,
R¹ Wasserstoff oder eine C₁- bis C₅-Alkylgruppe ist,
R² Wasserstoff oder eine C₁- bis C₅-Alkylgruppe ist,
n 2 oder 3 ist.
zur Herstellung eines Dentalmaterials gemäß einem der Ansprüche 1 bis 11.

13. Verwendung nach Anspruch 12 als dentales Adhäsiv, dentales Beschichtungsmaterial, dentales Füllungsmaterial oder dentaler Zement.

## Claims

1. Dental material comprising an amide of the general formula BXₙ in which
B stands for a hydrocarbon group containing 2 to 50 carbon atoms which can comprise one or more of the groups O, S, NH, CO-NH, O-CO-NH and/or NH-CO-NH, and which is substituted n times with the group X, wherein B is a saturated, linear or branched aliphatic group containing 2 to 15 carbon atoms which can contain one or two of the groups S, NH, O, NH-CO-O or O-CO-NH,
a cycloaliphatic group containing 6 or 15 carbon atoms,
an aromatic or non-aromatic heterocyclic group containing 3 to 10 carbon atoms and 1 to 3 heteroatoms,
an aromatic group containing 6 to 12 carbon atoms or
a combination of these groups,
X stands for the group 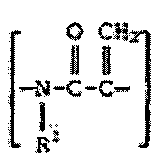 which is bound to the group B through the nitrogen atom or through C-2, wherein the binding site not connected to B carries a group R²,
R¹ is hydrogen or a C₁ to C₅ alkyl group,
R² is hydrogen or a C₁ to C₅ alkyl group,
n is 2 or 3, and
at least one polymerisable acidic monomer and a polymerisation initiator.

2. Dental material according to claim 1, **characterised in that** B carries, in addition to the group X, one or more substituents that are selected from Cl, Br, OH and/or COOH.

3. Dental material according to one of claims 1 to 2, **characterized in that** R¹ and/or R² are mono or polysubstituted, wherein the substituent or substituents are selected from Cl, Br, OH and/or COOH.

4. Dental material according to one of claims 1 to 3 comprising a polymerisable binder.

5. Dental material according to claim 3 or 4 comprising at least one ethylenically unsaturated polymerisable monomer.

6. Dental material according to claim 5 comprising a polyfunctional polymerisable monomer.

7. Dental material according to one of claims 3 to 6, **characterized in that** the quantity of the amide BXₙ relative to the sum of the masses of the amide BXₙ and other polymerisable monomers is more than 3 wt %, preferably more than 10 wt %.

8. Dental material according to one of claims 3 to 7 comprising an initiator for the photopolymerisation.

9. Dental material according to one of claims 1 to 8 comprising filler.

10. Dental material according to one of claims 1 to 9 comprising at least 1 wt %, preferably at least 5 wt %, of the amide BXₙ relative to the overall mass of the dental material.

11. Dental material according to one of claims 1 to 4 comprising
(a) 1 to 90 wt % of the amide BXₙ,
(b) 0.1 to 5.0 wt % polymerisation initiator,
(c) 0 to 70 wt % polymerisable monomer (non-acidic),
(d) > 0 to 70 wt % polymerisable acidic monomer,
(e) 0 to 70 wt % filler,
(f) 0 to 70 wt % solvent
in each case relative to the total mass of the dental material

12. Use of an amide of the general formula BXₙ in which
B stands for a hydrocarbon group containing 2 to 50 carbon atoms which can comprise one or more of the groups O, S, NH, CO-NH, O-CO-NH and/or NH-CO-NH, and which is substituted n times by the group X, wherein B is a saturated, linear or branched aliphatic group containing 2 to 15 carbon atoms which can contain one or two of the groups S, NH, O, NH-CO-O or O-CO-NH,
a cycloaliphatic group containing 6 or 15 carbon atoms,
an aromatic or non-aromatic heterocyclic group containing 3 to 10 carbon atoms and 1 to 3 heteroatoms,
an aromatic group containing 6 to 12 carbon atoms or
a combination of these groups,
X stands for the group 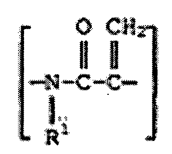 which is bonded to the group B through the nitrogen atom or through C-2, wherein the binding site not connected to B carries a group R²,
R¹ is hydrogen or a C₁ to C₅ alkyl group,
R² is hydrogen or a C₁ to C₅ alkyl group,
n is 2 or 3,
for the preparation of a dental material according to one of claims 1 to 11.

13. Use according to claim 12 as a dental adhesive, dental coating material, dental filling material or dental cement.

## Revendications

1. Matériau dentaire contenant un amide de formule générale
BXₙ
dans laquelle
- B représente un reste d'hydrocarbure comportant 2 à 50 atomes de carbone, porteur de n substituants symbolisés par X, qui peut comporter un ou plusieurs chaînons symbolisés par O, S, NH, CO-NH, O-CO-NH et/ou NH-CO-NH, lequel symbole B représente :
- un groupe aliphatique saturé, à chaîne linéaire ou ramifiée, comportant 2 à 15 atomes de carbone, qui peut comporter un ou deux chaînons symbolisés par S, NH, O, NH-CO-O ou O-CO-NH,
- un groupe cycloaliphatique comportant 6 ou 15 atomes de carbone,
- un groupe hétérocyclique aromatique ou non aromatique, comportant 3 à 10 atomes de carbone et 1 à 3 hétéroatomes,
- un groupe aromatique comportant 6 à 12 atomes de carbone,
- ou une combinaison de tels groupes ;
- X représente un groupe de formule 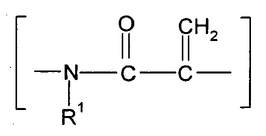 qui est lié au reste B par l'intermédiaire de l'atome d'azote ou de l'atome de carbone C2 et dont la position non occupée par le reste B porte un groupe représenté par R², étant entendu que
R¹ représente un atome d'hydrogène ou un groupe alkyle en C₁₋₅,
et R² représente un atome d'hydrogène ou un groupe alkyle en C₁₋₅ ;
- et l'indice n vaut 2 ou 3 ;
au moins un monomère polymérisable acide, et un amorceur de polymérisation.

2. Matériau dentaire conforme à la revendication 1, **caractérisé en ce que** le reste B porte, en plus des groupes X, un ou plusieurs substituant(s) choisi(s) parmi un atome de chlore, un atome de brome, un groupe hydroxyle et/ou un groupe carboxyle.

3. Matériau dentaire conforme à l'une des revendications 1 à 2, **caractérisé en ce que** les groupes R¹ et/ou R² portent un ou plusieurs substituant(s) choisi(s) parmi un atome de chlore, un atome de brome, un groupe hydroxyle et/ou un groupe carboxyle.

4. Matériau dentaire conforme à l'une des revendications 1 à 3, **caractérisé en ce qu'**il contient un liant polymérisable.

5. Matériau dentaire conforme à la revendication 3 ou 4, **caractérisé en ce qu'**il contient au moins un monomère polymérisable à insaturation éthylénique.

6. Matériau dentaire conforme à la revendication 5, **caractérisé en ce qu'**il contient un monomère polymérisable polyfonctionnel.

7. Matériau dentaire conforme à l'une des revendications 3 à 6, **caractérisé en ce que** la proportion de l'amide BXₙ, rapportée à la somme des masses de l'amide BXₙ et de tous les monomères polymérisables, vaut plus de 3 % en poids et de préférence plus de 10 % en poids.

8. Matériau dentaire conforme à l'une des revendications 3 à 7, **caractérisé en ce qu'**il contient un amorceur pour photopolymérisation.

9. Matériau dentaire conforme à l'une des revendications 1 à 8, **caractérisé en ce qu'**il contient une charge.

10. Matériau dentaire conforme à l'une des revendications 1 à 9, **caractérisé en ce qu'**il contient de l'amide BXₙ en une proportion d'au moins 1 % et de préférence d'au moins 5 %, en poids rapporté au poids total du matériau dentaire.

11. Matériau dentaire conforme à l'une des revendications 1 à 4 **caractérisé en ce qu'**il contient :
a) de 1 à 90 % en poids de l'amide BXₙ,
b) de 0,1 à 5,0 % en poids d'amorceur de polymérisation,
c) de 0 à 70 % en poids de monomère polymérisable non acide,
d) de plus de 0 à 70 % en poids de monomère polymérisable acide,
e) de 0 à 70 % en poids de charge,
f) et de 0 à 70 % en poids de solvant,
à chaque fois par rapport au poids total du matériau dentaire.

12. Utilisation d'un amide de formule générale
BXₙ
dans laquelle
- B représente un reste d'hydrocarbure comportant 2 à 50 atomes de carbone, porteur de n substituants symbolisés par X, qui peut comporter un ou plusieurs chaînons symbolisés par O, S, NH, CO-NH, O-CO-NH et/ou NH-CO-NH, lequel symbole B représente :
- un groupe aliphatique saturé, à chaîne linéaire ou ramifiée, comportant 2 à 15 atomes de carbone, qui peut comporter un ou deux chaînons symbolisés par S, NH, O, NH-CO-O ou O-CO-NH,
- un groupe cycloaliphatique comportant 6 ou 15 atomes de carbone,
- un groupe hétérocyclique aromatique ou non aromatique, comportant 3 à 10 atomes de carbone et 1 à 3 hétéroatomes,
- un groupe aromatique comportant 6 à 12 atomes de carbone,
- ou une combinaison de tels groupes ;
- X représente un groupe de formule 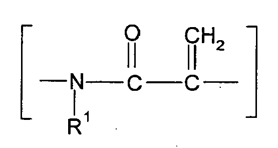 qui est lié au reste B par l'intermédiaire de l'atome d'azote ou de l'atome de carbone C2 et dont la position non occupée par le reste B porte un groupe représenté par R², étant entendu que
R¹ représente un atome d'hydrogène ou un groupe alkyle en C₁₋₅,
et R² représente un atome d'hydrogène ou un groupe alkyle en C₁₋₅ ;
- et l'indice n vaut 2 ou 3 ;
en vue de la fabrication d'un matériau dentaire conforme à l'une des revendications 1 à 11.

13. Utilisation conforme à la revendication 12, le matériau dentaire étant un adhésif dentaire, un matériau de revêtement dentaire, un matériau d'obturation dentaire ou un ciment dentaire.
